# EUROPÄISCHE PATENTSCHRIFT

(11) **EP 0 658 082 B1**
(45) Veröffentlichungstag und Bekanntmachung des Hinweises auf die Patenterteilung: **27.05.1998**
(21) Anmeldenummer: 93919122.7
(22) Anmeldetag: 20.08.1993
(51) Int. Cl.: A01N 63/02, A61K 7/32, A61K 7/48

(54) **GERMICIDE WIRKSTOFFKOMBINATIONEN**
COMBINATION OF GERMICIDAL AGENTS
COMBINAISONS DE MATIERES ACTIVES GERMICIDES

(30) Priorität: 05.09.1992 DE 4229707
(43) Veröffentlichungstag der Anmeldung: 21.06.1995
(73) Patentinhaber: Beiersdorf Aktiengesellschaft, 20245 Hamburg (DE)
(72) Erfinder: KLEIN, Winfried, D-22297 Hamburg (DE); MATTHIES, Eva, D-22453 Hamburg (DE); SAUERMANN, Gerhard, D-24649 Wiemersdorf (DE); SCHMIDT-LEWERKÜHNE, Hartmut, D-22869 Schenefeld (DE); SCHMUCKER, Robert, D-22457 Hamburg (DE); TRAUPE, Bernd, D-22457 Hamburg (DE)
(86) Internationale Anmeldenummer: EP9302238
(87) Internationale Veröffentlichungsnummer: WO9405156

(56) Entgegenhaltungen:
- EP-A- 0 274 267
- EP-A- 0 451 002
- WO-A-91/07164

## Beschreibung

Die vorliegende Erfindung betrifft Wirkstoffkombinationen zum Schutze gegen Mikroorganismen. Ferner betrifft sie Zubereitungen, vorzugsweise kosmetische und dermatologische Zubereitungen, insbesondere kosmetische Desodorantien, solche Wirkstoffkombinationen enthaltend. Weiterhin betrifft sie Verfahren zur Stabilisierung von Zubereitungen, insbesondere kosmetischen und dermatologischen Zubereitungen, gegen den Befall von Mikroorganismen.

Wirkstoffkombinationen zum Schutze gegen Mikroorganismen werden beispielsweise in Zubereitungen verwendet, welche auf die Haut oder Schleimhaut eines Menschen oder eines Tieres aufgetragen werden, um auf oder in der Haut befindlichen Mikroorganismenbefall zu vermindern, zu beseitigen oder gegen einen Mikroorganismenbefall vorzubeugen. Unter solche Mittel fallen begrifflich die topischen Dermatika und Kosmetika.

Allerdings wird im Rahmen der vorliegenden Erfindung auch der Schutz gegen Mikroorganismenbefall von Zubereitungen selbst, insbesondere von kosmetischen und dermatologischen Zubereitungen als Schutz gegen Mikroorganismen angesehen. In der Fachsprache wird dieser Schutz als Konservierung bezeichnet. Konservierung ist an sich nicht auf kosmetische oder dermatologische Zubereitungen beschränkt. Vielmehr bezieht sie sich auf den Schutz aller organischen Materialien gegen mikrobiellen Abbau.

Kosmetische Zubereitungen zum Schutze gegen Mikroorganismen sind in erster Linie Desodorantien, also Formulierungen, welche dazu dienen, Körpergeruch zu beseitigen. Körpergeruch entsteht, wenn der an sich geruchlose frische Schweiß durch Mikroorganismen zersetzt wird. Dabei entsteht eine große Anzahl leichtflüchtiger Substanzen, beispielsweise nur genannt die übelriechende iso-Valeriansäure.

### Desodorantien:

Den kosmetischen Desodorantien des Standes der Technik liegen unterschiedliche Wirkprinzipien zugrunde.

Durch Adstringentien - vorwiegend im Gebrauch sind Aluminiumsalze wie Aluminiumhydroxychlorid - kann die Entstehung des Schweißes unterbunden werden. Abgesehen davon, daß die damit verbundene Denaturierung der Hautproteine eine unerwünschte Nebenreaktion darstellt, greifen die dafür verwendeten Stoffe zudem in den Wärmehaushalt der Haut ein und sollten allenfalls in Ausnahmefällen angewandt werden.

Durch antimikrobielle Stoffe kann die Bakterienflora auf der Haut reduziert werden. Dabei sollten im Idealfalle nur die Geruch verursachenden Mikroorganismen vernichtet werden. In der Praxis hat sich aber herausgestellt, daß die gesamte Mikroflora der Haut in gleichem Maße geschädigt wird. Gelegentlich werden sogar die Mikroorganismen, die keinen Geruch verursachen, stärker geschädigt.

Schließlich kann Körpergeruch auch durch Duftstoffe überdeckt werden, die klassische Methode, die aber am wenigsten den ästhetischen Bedürfnissen des Verbrauchers gerecht wird, da die Mischung aus Körpergeruch und Parfümduft eher unangenehm riecht.

Desodorantien sollen folgende Bedingungen erfüllen:
(1) Die biologischen Vorgänge der Haut dürfen nicht beeinträchtigt werden
(2) Die Desodorantien sollen keinen ausgeprägten Eigengeruch besitzen
(3) Sie müssen bei Überdosierung oder sonstiger nicht bestimmungsgemäßer Anwendung unschädlich sein.
(4) Sie sollen sich nach wiederholter Anwendung nicht auf der Haut anreichern.
(5) Sie sollen sich gut in handelsübliche kosmetische Formulierungen einarbeiten lassen.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays Intimreinigungsmittel usw.

Eine Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Mikroorganismen aber, die für den Körpergeruch verantwortlich sind, selektiv reduzieren.

### Topische Dermatika:

Einige der unangenehmen oder pathogenen Keime befallen die verschiedenen Schichten der Haut, darunter der Akneerreger Propionibacterium acnes. P.acnes besiedelt bevorzugt die Haarfollikel und kommt Zumeist im Pubertätsintervall der betroffenen Personen zum Ausbruch. Die mit ihm verbundenen, oft erheblichen Hautläsionen sind bestenfalls unschön, können den Patienten aber auch seelisch schwer belasten.

Auch Lantibiotika sind bereits als Therapeutika gegen mikrobiell verursachte Hautleiden, namentlich Akne, vorgeschlagen worden. Nachteilig ist, daß diese Zubereitungen nur kurzzeitig stabil sind.

Eine weitere Aufgabe der vorliegenden Erfindung war also, Zubereitungen und Verfahren zur Verfügung zu stellen, welche diesem Mißstande abhelfen.

### Stabilisierung von parfümierten kosmetischen Zubereitungen:

Aus der DE-OS 39 38 140 sind kosmetische Zubereitungen, nämlich Desodorantien, mit einem wirksamen Gehalt an Lantibiotika bekannt. Die dort beschriebenen Zusammensetzungen zeichnen sich durch hervorragende Wirkung und Verträglichkeit aus.

Die dort beschriebenen kosmetischen Desodorantien haben jedoch den Nachteil, daß auf bisher noch nicht erschöpfend geklärte Weise ein Zusatz an Parfümbestandteilen die desodorierende Wirksamkeit der Lantibiotika in unerwünschter Weise herabsetzt. Da fast alle Kosmetika, ob desodorierend oder nicht, Parfümbestandteile enthalten, stellt diese Wirkungsminderung ein erhebliches Problem dar.

Eine weitere Aufgabe der Erfindung bestand also darin, die schädliche Einwirkung von Parfümbestandteilen auf Wirkstoffkomponenten in kosmetischen Zubereitungen zu unterbinden.

### Darlegung der Erfindung:

Es war erstaunlich und für den Fachmann nicht vorhersehbar, und darin liegt die Lösung all dieser Aufgaben begründet, daß

Kombinationen aus Imidazol und/oder einem oder mehreren Derivaten des Imidazols, welche folgende allgemeine Strukturformel haben können 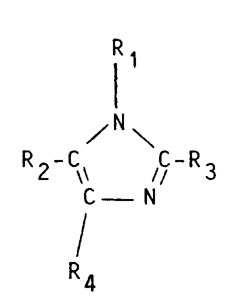
- wobei die Reste folgende Bedeutung haben können:
   R₁ = H, C_{(1 - 25)} - Alkyl,
   R₂ = H, C_{(1 - 25)} - Alkyl,
   R₃ = H, C_{(1 - 25)} - Alkyl,
   R₄ = H, C_{(1 - 25)} - Alkyl bedeuten,
- wobei R₄, auch, und zwar bevorzugt dann, wenn R₁, R₂ und R₃ ein Wasserstoffatom darstellen, die Reste

   -CH₂-CH₂-NR₅R₆ und -CH₂-CH(NR₅R₆)-COOR₇

   repräsentieren kann, wobei
   R₅ = H, C_{(1 - 25)} - Alkyl,
   R₆ = H, C_{(1 - 25)} - Alkyl,
   R₇ = H, C_{(1 - 25)} - Alkyl bedeuten,
   und einem Lantibiotikum oder einem Gemisch aus mehreren Lantibiotika
- bei der Lagerung stabil sein würden,
- eine genügend hohe Halbwertszeit auf der Haut besitzen würden,
- zur Anwendung als Kosmetikum geeignet sein würden,
- Wechselwirkungen zwischen Lantibiotika und Parfümbestandteilen nicht auftreten lassen
- bei der Anwendung in Kosmetika die geeignete Stabilität aufweisen würden,
- selektiv gegen gerucherzeugende Mikroorganismen wirksam sein würden,
- die symbiontische Mikroflora der Haut schonen würden,
- auch bei der Verwendung von Parfümkomponenten voll aktiv sein würden
- eine deutliche Verbesserung der Deowirkung gegenüber den imidazolfreien Zusammensetzungen erzielen würden,
- bei der Anwendung als Konservierungsmittel die geeignete Stabilität aufweisen würden,
- bei der Anwendung in Lebensmitteln die geeignete Stabilität aufweisen würden,

Insbesondere war erstaunlich, daß die erfindungsgemäßen Zusammensetzungen nicht nur für kosmetische Zwecke geeignet sind, sondern überdies wirkungsvoller und schonender sind als die Zusammensetzungen des Standes der Technik.

Lantibiotika an sich sind seit vielen Jahren bekannt. Es sind Polypeptide, die von Mikroorganismen synthetisiert werden und sich durch Vertreter der Aminosäuregruppe der Lanthionine als Strukturelement in der Peptidsequenz auszeichnen.

Die Lanthionine haben folgende Strukturen:
HOOC-CH(NH₂)-CH₂-S-CH₂-CH(NH₂)-COOH
andere Schreibweise: 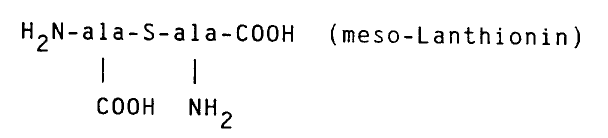 sowie
HOOC-CH(NH₂)-CH₂-S-CH(CH₃)-CH(NH₂)-COOH
andere Schreibweise:

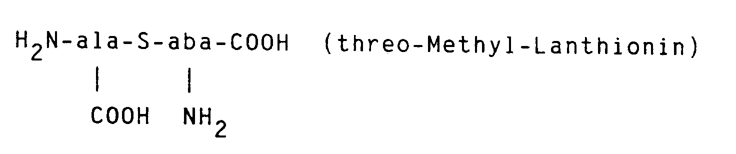

Mit diesen Lanthioninen werden in den Lantibiotika Ringstrukturen gebildet.

Beispiele für Lantibiotika sind Nisin, Epidermin, Subtilin, Cinramycin, Duramycin, Ancovenin, Gallidermin, Pep 5.

Die vorgenannten Stoffe sind an sich bekannt und können unter den Registraturnummern der Chemical Abstracts ermittelt werden:
- Nisin: 1414-45-5
- Epidermin: 99165-17-0
- Subtilin: 1393-38-0
- Pep 5: 110655-58-8
- Duramycin: 1391-36-2
- Ancovenin: 88201-41-6
- Gallidermin: 117978-77-5

Nisin beispielsweise ist ein Peptid aus 34 Aminosäuren, welches von Streptococcus lactis synthetisiert wird.

Nisin besitzt folgende Aminosäurensequenz (Primärstruktur): 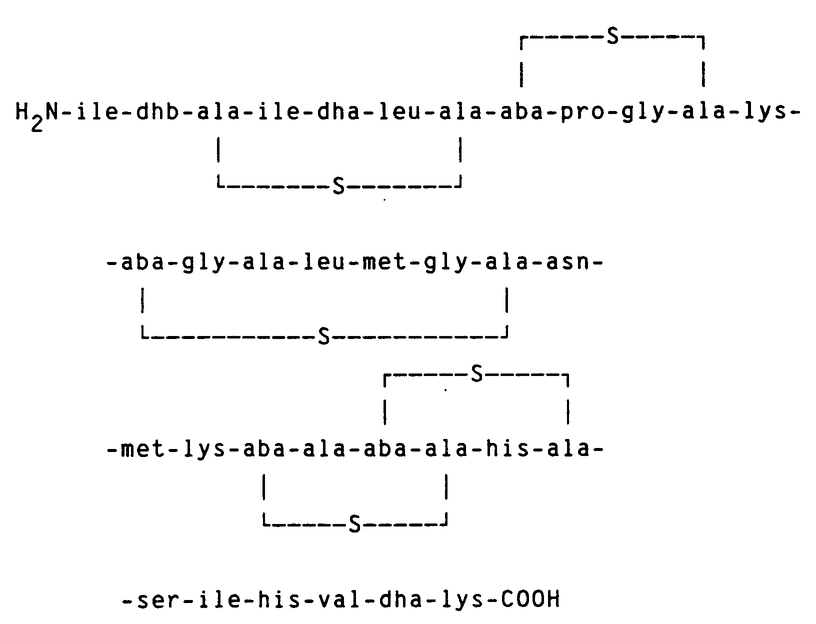 wobei
dhb = Dehydrobutyrin
dha = Dehydroalanin
aba = Aminobuttersäure

E.Gross, J.L.Morell , "The Structure of Nisin" , J.Amer.Chem.Soc. 93, Ss. 4634-4637 (1971).

Der Wirkungsmechanismus von Nisin und den mit Nisin verwandten und im wesentlichen wirkungsgleichen Lantibiotika kann wie folgt gedeutet werden: Durch Freisetzung von Autolysinen werden Zellwände zerstört. Da zudem in der Cytoplasmamembran Kanäle gebildet werden, können niedermolekulare Zellbestandteile ausdiffundieren, wodurch die (prokaryotische) Zelle vernichtet wird.

Die Richtigkeit dieser Deutung ist indes für die Erfindung ohne Belang. Es soll nur der Versuch unternommen werden, die mikrobiologischen Vorgänge zu erläutern.

Eukaryotische Zellen, also Hautzellen, Pilze usw, sind gegen Nisin und andere Lantibiotika resistent.

Lantibiotika sind für den Warmblüter praktisch ungiftig. Die LD50 für Nisin beispielsweise ist größer als 7 g/kg (ermittelt für Ratten und Katzen). Es ist bekannt, daß Nisin sowie die anderen Lantibiotika vorwiegend gegen Micrococcen und coryneforme Bakterien wirken. In manchen Ländern, vorwiegend in Osteuropa, ist es ein als Lebensmittelkonservierungsstoff zugelassener Stoff (nicht in Deutschland).

Lantibiotika werden aus traditionellen Gründen oft unter die sogenannten "Peptidantibiotika" gerechnet. Sie sind jedoch aus mancherlei Gründen nicht als klassische Antibiotika aufzufassen, zumindest nicht alle ihre Vertreter, am wenigsten das Nisin. Alles spricht eher dafür, sie mit der Bezeichnung "Bacteriocine" zu versehen. Bacteriocine sind von Bakterien produzierte Proteine, die verwandte Bakterienarten oder -stämme töten oder in ihrem Wachstum hemmen. Sie werden meist von Plasmiden codiert, den sogenannten Bacteriocinfaktoren.

Epidermin ist ein Lantibiotikum das aus auf der menschlichen Haut vorkommenden Mikroorganismen isoliert wurde. Die Verwendung von Epidermin als topisch wirksames Antibiotikum/Therapeutikum wird in EP-A-0 181 578 beschrieben. Es wird zur Bekämpfung infektiöser Erkrankungen verwendet.

Gallidermin ist ein Lantibiotikum, welches sich nur durch eine Aminosäure von Epidermin unterscheidet. In EP-A-0 342 486 wird ein kosmetisches Mittel, enthaltend Gallidermin zitiert, ohne daß aber Kosmetika offenbart würden, und ohne daß die vorteilhafte Kombination aus Lantibiotika und Imidazol oder dessen Abkömmlingen nahegelegt würden.

Bekannt ist ferner aus der internationalen Patentanmeldung WO 89/12399, Kombinationen aus Nisin und Komplexanden und/oder Emulgatoren als Konservierungsmittel zu verwenden.

Imidazol ist durch die Strukturformel 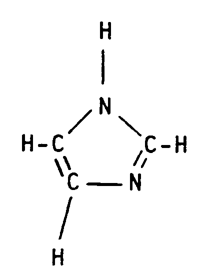 gekennzeichnet. Imidazol ist ebenfalls für den Warmblüter praktisch ungiftig, wirkt allerdings bei niederen Tieren als Antimetabolit gegen Histamin und Nicotinsäure. Gewisse Derivate des Imidazols werden als Antimykotika verwendet.

Derivate des Imidazols, welche erfindungsgemäß ebenfalls verwendet werden können, sind solche der allgemeinen Strukturformel 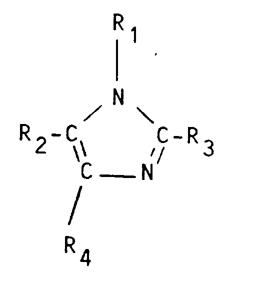 wobei die Reste folgende Bedeutung haben können:
R₁ = H, C_{(1 - 25)} - Alkyl,
R₂ = H, C_{(1 - 25)} - Alkyl,
R₃ = H, C_{(1 - 25)} - Alkyl,
R₄ = H, C_{(1 - 25)} - Alkyl bedeuten,
wobei R₄, auch, und zwar bevorzugt dann, wenn R₁, R₂ und R₃ ein Wasserstoffatom darstellen, die Reste

-CH₂-CH₂-NR₅R₆ und -CH₂-CH(NR₅R₆)-COOR₇

repräsentieren kann, wobei
R₅ = H, C_{(1 - 25)} - Alkyl,
R₆ = H, C_{(1 - 25)} - Alkyl,
R₇ = H, C_{(1 - 25)} - Alkyl bedeuten,

Unter diesen Derivaten des Imidazols sind diejenigen bevorzugt, welche wasser- und/oder alkohollöslich sind. Besonders bevorzugt sind jene Derivate, bei welchen R₁ = H ist.

Insbesondere ist vorteilhaft, den underivatisierten Grundkörper (R₁ - R₄ = H) des Imidazols zu verwenden.

Vorteilhaft sind auch die Derivate Histidin 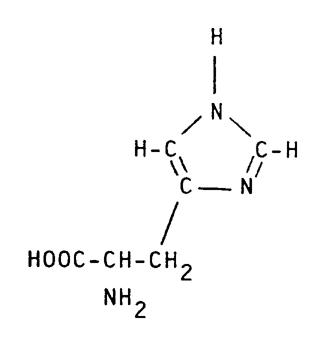 und Histamin,

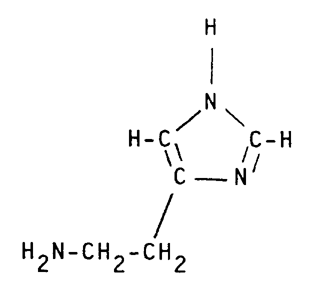

Die Wirksamkeit ausgewählter erfindungsgemäßer Zusammensetzungen nimmt in der folgenden Reihenfolge ab:
Imidazol (unsubstituiert) > Histamin > Histidin

Natürlich ist dem Fachmann klar, daß die Verwendung von Histamin in Produkten, die in unmittelbaren Kontakt mit dem menschlichen oder tierischen Organismus, also etwa Kosmetika und Lebensmittel, nicht unbedenklich wäre, da Histamin als Gewebshormon unerwünschte Reaktionen zeitigen kann: Histamin tritt vermehrt bei Allergien und Anaphylaxien auf.

Die Verwendung der erfindungsgemäßen Kombination aus Histamin und Lantibiotika sollte daher der ärztlichen Aufsicht unterliegen.

Die erfindungsgemäßen Kombination sind vorteilhaft durch einen Gehalt von
- 0,01 - 99,99 Gew.-%: einer Imidazolkomponente und
- 99,99 - 0,01 Gew.-%: eines Einzelstoffes oder eines Gemisches aus der Gruppe der Lantibiotika,
bezogen auf das Gesamtgewicht der Kombination, gekennzeichnet.

Die erfindungsgemäßen Kombination sind insbesondere vorteilhaft durch einen Gehalt von
- 0,1 - 99,9 Gew.-%: einer Imidazolkomponente und
- 99,9 - 0,1 Gew.-%: eines Einzelstoffes oder eines Gemisches aus der Gruppe der Lantibiotika,
bezogen auf das Gesamtgewicht der Kombination, gekennzeichnet.

Die erfindungsgemäßen Kombination sind ganz besonders vorteilhaft durch einen Gehalt von
- 1,0 - 99,0 Gew.-%: einer Imidazolkomponente und
- 99,0 - 1,0 Gew.-%: eines Einzelstoffes oder eines Gemisches aus der Gruppe der Lantibiotika,
bezogen auf das Gesamtgewicht der Kombination, gekennzeichnet.

In den endgültigen Zubereitungen liegen die Lantibiotika bevorzugt in Konzentrationen von 0,1 - 10000 ppm vor. Besonders bevorzugt liegen die Lantibiotika in den endgültigen Zubereitungen in Konzentrationen von 0,1 - 750 ppm, ganz besonders bevorzugt in Konzentrationen von 0,5 - 400 ppm, vor. Die Konzentrationsangaben beziehen sich jeweils auf den Gehalt an reinem Wirkstoff und auf das Gesamtgewicht der Zusammensetzung.

Als besonders vorteilhaft haben sich Formulierungen mit einem wirksamen Gehalt an Nisin, Epidermin und/oder Gallidermin herausgestellt. Aber auch die anderen genannten Lantibiotika sind gut für die erfindungsgemäße Verwendung geeignet.

Ganz besonders vorteilhaft ist es, Nisin zu verwenden.

Es ist möglich und gegebenenfalls vorteilhaft, ein Gemisch an Lantibiotika als wirksames Prinzip der erfindungsgemäßen Zusammensetzungen einzusetzen.

Es ist besonders vorteilhaft, die erfindungsgemäßen Zusammensetzungen auf einen pH-Bereich von 2,5 - 6,5 abzupuffern. Besonders günstig ist es, den pH-Wert in einem Bereich von 3,5 - 4,8 zu wählen.

Die Konzentrationen an Imidazol betragen vorteilhaft 0,01 - 5,00 Gew.-%, bezogen auf das Gesamtgewicht der Zusammensetzung, bevorzugt 0,10 - 2,00 Gew.-%, ganz besonders bevorzugt 0,25 - 1,00 Gew.-%

Insbesondere sind Zusammensetzungen vorteilhaft, in welchen Kombinationen mit einem Gehalt von
- 0,1 - 10000 ppm: Lantibiotika, insbesondere Nisin, und
- 0,01 - 5,00 Gew.-%: Imidazol,
bevorzugt
- 2,0 - 750 ppm: Lantibiotika, insbesondere Nisin, und
- 0,10 - 2,00 Gew.-%: Imidazol,
besonders bevorzugt
- 5,0 - 400 ppm: Lantibiotika, insbesondere Nisin, und
- 0,25 - 1,00 Gew.-%: Imidazol,
jeweils bezogen auf das Gesamtgewicht der Zubereitung, enthalten sind.

Insbesondere bevorzugt sind solche Formulierungen, welche 0,4 - 0,6 Gew.-% Imidazol oder dessen Derivate enthalten.

Parfümöle, die insbesondere Lantibiotika nachhaltig desaktivieren, sind in Tabelle 1 aufgeführt. Liegen aber Lantibiotika in Kombination mit Imidazol oder dessen Derivaten vor, so ist die Destabilisierung kompensiert.

**Tabelle 1**

| | |
|---|---|
| Aldehyd C12 | Hydroxycitronellol |
| Allylamylglycolat | Isoeugenol |
| Ambrettolid | Isoraldein 70 |
| Amylzimtaldehyd | Jonol |
| Anisaldehyd | Lilial (Firmenich) |
| Aurantesin | Lyral |
| Benzylacetat | Methyldihydrojasmonat |
| Benzylsalicylat | Moschusketon |
| Castoreum | p-Hydroxybenzylaceton |
| Cedrylketon | Patchouliöl |
| Eugenol | Phenylethylalkohol |
| Florol (Firmenich) | Phenylethylphenylacetat |
| Gamma Decalacton | Phenylethylprivalat |
| Geraniol | Tagetesöl |
| Geraniumöl Bourbon | Terpentinöl |
| Geranylacetat | Vertosine |
| Helosine | Ylang-Ylang-Öl |
| Hexenylsalicylat | Zibeth |
| Hydrocarboresin | Zimtaldehyd |
| Hydroxycitronellal (Firmenich) | Zimtalkohol |

Außer den anders gekennzeichneten Parfümöl werden die in Tabelle 1 aufgeführten Parfümöle von der Firma Haarmann & Reimer vertrieben.

Die Stabilisierung gegenüber den in Tabelle 1 genannten Substanzen wird erfindungsgemäß beansprucht, soll aber nicht darauf beschränkt sein.

Erfindungsgemäß ist also auch die Verwendung eines oder mehrere Derivate des Imidazols, welche folgende allgemeine Strukturformel aufweisen 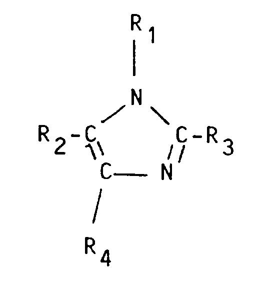
- wobei die Reste folgende Bedeutung haben können:
   R₁ = H, C_{(1 - 25)} - Alkyl,
   R₂ = H, C_{(1 - 25)} - Alkyl,
   R₃ = H, C_{(1 - 25)} - Alkyl,
   R₄ = H, C_{(1 - 25)} - Alkyl bedeuten,
- wobei R₄, auch, und zwar bevorzugt dann, wenn R₁, R₂ und R₃ ein Wasserstoffatom darstellen, die Reste

   -CH₂-CH₂-NR₅R₆ und -CH₂-CH(NR₅R₆)-COOR₇

   repräsentieren kann, wobei
   R₅ = H, C_{(1 - 25)} - Alkyl,
   R₆ = H, C_{(1 - 25)} - Alkyl,
   R₇ = H, C_{(1 - 25)} - Alkyl bedeuten,
zur Stabilisierung von Lantibiotika.

Die desodorierenden kosmetischen Mittel gemäß der Erfindung kann in Form eines aus Aerosolbehältern, Quetschflaschen oder durch eine Pumpvorrichtung versprühbaren Präparates vorliegen oder in Form einer mittels Roll-on-Vorrichtungen auftragbaren flüssigen Zusammensetzung, jedoch auch in Form einer aus normalen Flaschen und Behältern auftragbaren W/O- oder O/W-Emulsion, z.B. einer Creme oder Lotion. Weitere kosmetische Desodorantien können in Form von desodorierenden Tinkturen, desodorierenden Intimreinigungsmittel, desodorierenden Shampoos, Deo-Seife, desodorierenden Dusch- oder Badezubereitungen, desodorierenden Pudern oder desodorierenden Pudersprays vorliegen. Es können aber auch übliche Deo-Stift-Grundmassen als Träger für feste Zubereitungen und Stifte dienen.

Als übliche kosmetische Trägerstoffe zur Herstellung der erfindungsgemäßen desodorierenden Mittel können neben Wasser, Äthanol und Isopropanol, Glycerin und Propylenglykol hautpflegende Fett- oder fettähnliche Stoffe, wie Partialglyceride von Fettsäuregemischen, Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Methylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Als Treibmittel für aus Aerosolbehältern in Form eines Sprühstrahls bei Betätigung des Ventils versprühbare desodorierende kosmetische Mittel gemäß der Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist gegebenenfalls vorteilhaft zu verwenden.

Als Emulgatoren zur Herstellung der erfindungsgemäßen desodorierenden kosmetischen Mittel, die vorzugsweise als flüssige Zubereitungen mittels einer Roll-on-Vorrichtung auf die gewünschten Hautbereiche aufgetragen werden sollen, und die in den Mitteln in geringer Menge (z.B.) 2 bis 5 Gewichts.-%, bezogen auf die Gesamt-Zusammensetzung, verwendet werden können, haben sich nichtionogene Typen, wie Polyoxyäthylen-Fettalkoholäther, z.B. Cetostearylalkoholpolyäthylenglykoläther mit 12 bzw. 20 angelagerten Äthylenoxid-Einheiten pro Molekül Cetostearylalkohol, sowie Sorbitanester und Sorbitanester-Äthylenoxid-Verbindungen (z.B. Sorbitanmonostearat und Polyoxyäthylensorbitanmonostearat) als auch langkettige höhermolekulare wachsartige Polyglykoläther als geeignet erwiesen.

Zusätzlich zu den genannten Bestandteilen können den desodorierenden kosmetischen Mitteln gemäß der Erfindung, deren pH-Wert vorzugsweise z.B. durch übliche Puffergemische auf 2,5 bis 6,5, insbesondere 3,5 bis 4,8, eingestellt wird, Parfüm, Farbstoffe, Antioxidantien (z.B. alpha-Tocopherol oder Butylhadroxytoluol ( = 2,6-Di-Tert.-butyl-4-methylphenol in Mengen von 0,01 bis 0,03%, bezogen auf die Gesamt-Zusammensetzung), Suspendiermittel, Puffergemische oder andere übliche kosmetische Grundstoffe, wie Triäthanolamin oder Harnstoff, beigemischt werden.

Zur Parfümierung sind gegebenenfalls auch solche Substanzen und Parfümöle geeignet, die stabil sind, die Haut nicht reizen und bereits als solche antibakterielle (bakteriostatische) Eigenschaften besitzen.

Es ist jedoch im Einzelfalle abzuwägen, ob der Vorteil, den die hochselektive antimikrobielle Wirkung der Lantibiotika und insbesondere der erfindungsgemäßen Kombinationen bietet, durch unter Umständen geringerselektive Wirkung anderer Stoffe gemindert werden soll, oder nicht.

Die Erfindung ist aber nicht auf die genannten Zubereitungen und Mittel, Hilfsstoffe und Trägersubstanzen beschränkt.

Die jeweils einzusetzenden Mengen an kosmetischen Trägerstoffen und Parfüm können in Abhängigkeit von der Art des jeweiligen Produktes vom Fachmann durch einfaches Ausprobieren leicht ermittelt werden.

Die Herstellung der kosmetischen Mittel erfolgt abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat gegebenenfalls unter Erwärmen hergestellt und dann emulgiert.

Ansonsten sind die üblichen Maßregeln für das Zusammenstellen von kosmetischen Formulierungen zu beachten, die dem Fachmann geläufig sind.

Die erfindungsgemäßen Kombinationen können auf einfache Weise in die erfindungsgemäßen Zusammensetzungen eingearbeitet werden. Bevorzugt werden sie in gelöster Form (z.B. als wäßrige, alkoholische oder alkoholisch-wäßrige Lösung) den übrigen Bestandteilen der Formulierungen zugesetzt. Ganz besonders vorteilhaft aber ist es, Lantibiotika in sogenannte Pudersprays einzuarbeiten. Vorteilhaft ist es auch, Zusatzstoffe zu vermeiden, welche die natürliche Mikroflora schädigen können, da die erfindungsgemäßen Kombinationen für sich selektiv die geruchserzeugenden Mikroorganismen reduzieren.

Sollen die erfindungsgemäßen Kombinationen in Pudersprays eingearbeitet werden, so können die Suspensionsgrundlagen dafür vorteilhaft gewählt werden aus der Gruppe
Aerosil, Kieselgur, Kaolin, Talkum, modifizierte Stärke, Titandioxid, Zinkoxid, Seidenpulver, Nylonpulver, Polyethylenpulver und verwandten Stoffen.

Die folgenden Beispiele dienen dazu, die Erfindung zu beschreiben, ohne daß beabsichtigt ist, die Erfindung auf diese Beispiele zu beschränken.

### Parfümkompositionen:

| Parfüm Nr. 1 | |
|---|---|
| Benzylacetat | 4,0 Gew.-% |
| Geraniol | 5,0 Gew.-% |
| Phenylethylalkohol | 9,0 Gew.-% |
| diverse andere Riechstoffe | ad 100,0 Gew.-% |

| Parfüm Nr. 2 | |
|---|---|
| Benzylacetat | 5,4 Gew.-% |
| Geraniol | 8,0 Gew.-% |
| Ylang-Ylang-Öl | 8,4 Gew.-% |
| diverse andere Riechstoffe | ad 100,0 Gew.-% |

| Parfüm Nr. 1 | |
|---|---|
| Benzylacetat | 5,0 Gew.-% |
| Geraniol | 12,0 Gew.-% |
| Hydroxycitronellal | 7,0 Gew.-% |
| diverse andere Riechstoffe | ad 100,0 Gew.-% |

| Beispiel 1 | |
|---|---|
| Pumpspray | |
| Nisin (reine Substanz) | 0,025 g |
| Imidazol | 5,000 g |
| Ethanol pharm. (96 %) | 354,875 g |
| Parfüm 1 | 10,000 g |
| Farbstoff | nach Belieben |
| Wasser | auf 1 000,000 g |

| Beispiel 2 | |
|---|---|
| Pumpspray | |
| Epidermin | 0,010 g |
| Imidazol | 3,500 g |
| Ethanol pharm. (96 %) | 354,875 g |
| Parfüm 1 | 10,000 g |
| Farbstoff | nach Belieben |
| Wasser | auf 1 000,000 g |

| Beispiel 3 | |
|---|---|
| Deo-Roller (Roll-on) | |
| Nisin | 0,150 g |
| Imidazol | 8,000 g |
| Hydroxyethylcellulose | 5,000 g |
| Propylenglycol | 5,000 g |
| Ethanol pharm. (96 %) | 355,850 g |
| Parfüm 2 | 10,000 g |
| Farbstoff | nach Belieben |
| Wasser | auf 1 000,000 g |

| Beispiel 4 | |
|---|---|
| Deo-Roller (Roll-on) | |
| Epidermin | 0,120 g |
| Imidazol | 6,500 g |
| Hydroxyethylcellulose | 5,000 g |
| Propylenglycol | 5,000 g |
| Ethanol pharm. (96 %) | 355,850 g |
| Parfüm 2 | 10,000 g |
| Farbstoff | nach Belieben |
| Wasser | auf 1 000,000 g |

| Beispiel 5 | |
|---|---|
| Spray | |
| Nisin | 0,200 g |
| Imidazol | 1,500 g |
| Ethanol pharm. (96 %) | 150,000 g |
| Propylenglycol | 50,000 g |
| Dimethylether | 300,000 g |
| Parfüm 3 | 10,000 g |
| Wasser | auf 1 000,000 g |

| Beispiel 6 | |
|---|---|
| Spray | |
| Epidermin | 0,150 g |
| Imidazol | 2,000 g |
| Ethanol pharm. (96 %) | 150,000 g |
| Propylenglycol | 50,000 g |
| Dimethylether | 300,000 g |
| Parfüm 3 | 10,000 g |
| Wasser | auf 1 000,000 g |

| Beispiel 7 | |
|---|---|
| Spray | |
| Nisin | 0,180 g |
| Imidazol | 17,000 g |
| Ethanol pharm. (96 %) | 497,160 g |
| 2-Octyldodecanol | 2,660 g |
| Parfüm 1 | 10,000 g |
| Dimethylether | auf 1 000,000 g |

| Beispiel 8 | |
|---|---|
| Spray | |
| Epidermin | 0,150 g |
| Imidazol | 13,800 g |
| Ethanol pharm. (96 %) | 497,160 g |
| 2-Octyldodecanol | 2,660 g |
| Parfüm 1 | 10,000 g |
| Dimethylether | auf 1 000,000 g |

| Beispiel 9 | |
|---|---|
| Nisin | 1,200 g |
| Imidazol | 32,000 g |
| Cetylstearylalkohol | 20,000 g |
| 2-Octyldodecanol | 20,000 g |
| Kaolin | 200,200 g |
| Talkum | 200,200 g |
| Aerosil | 48,200 g |
| Parfüm 3 | 10,000 g |
| Reisstärke | auf 1 000,000 g |

| Beispiel 10 | |
|---|---|
| Epidermin | 1,000 g |
| Imidazol | 27,000 g |
| Cetylstearylalkohol | 20,000 g |
| 2-Octyldodecanol | 20,000 g |
| Kaolin | 200,200 g |
| Talkum | 200,200 g |
| Aerosil | 48,200 g |
| Parfüm 3 | 10,000 g |
| Reisstärke | auf 1 000,000 g |

| Beispiel 11 | |
|---|---|
| Waschgelkonzentrat | |
| Nisin | 9,000 g |
| Imidazol | 17,000 g |
| Cocoamidopropylbetain | 613,300 g |
| Tipa-Laurylethersulfat | 306,700 g |
| Kochsalz | nach Belieben |
| Parfüm 2 | 40,000 g |
| Farbstoff | nach Belieben |
| Citronensäure | 1,000 g |
| Glycerin | 10,000 g |
| Wasser | auf 1 000,000 g |

| Beispiel 12 | |
|---|---|
| Waschgelkonzentrat | |
| Epidermin | 7,000 g |
| Imidazol | 12,000 g |
| Cocoamidopropylbetain | 613,300 g |
| Tipa-Laurylethersulfat | 306,700 g |
| Kochsalz | nach Belieben |
| Parfüm 12 | 40,000 g |
| Farbstoff | nach Belieben |
| Citronensäure | 1,000 g |
| Glycerin | 10,000 g |
| Wasser | auf 1 000,000 g |

| Beispiel 13 | |
|---|---|
| Puderspray I | |
| a) Suspensionsgrundlage | |
| Polymethylsiloxan | |
| (Cyclomethicone) | 72,000 g |
| Talkum | 24,000 g |
| Bentonitgel IPM | 3,000 g |
| Nisin | 1,000 g |
| Imidazol | 5,000 g |
| Parfüm 1 | 2,500 g |

| b) fertiger Spray | |
|---|---|
| Suspensionsgrundlage I | 20,000 g |
| Propan/Butan | 80,000 g |

| Beispiel 14 | |
|---|---|
| Puderspray II | |
| a) Suspensionsgrundlage | |
| Polymethylsiloxan (Cyclomethicone) | 72,000 g |
| Titandioxid | 24,000 g |
| Bentonitgel IPM | 3,000 g |
| Nisin | 1,000 g |
| Imidazol | 8,000 g |
| Parfüm 3 | 2,000 g |

| b) fertiger Spray | |
|---|---|
| Suspensionsgrundlage I | 20,000 g |
| Propan/Butan | 80,000 g |

| Beispiel 15 | |
|---|---|
| Puderspray III | |
| a) Suspensionsgrundlage | |
| Polymethylsiloxan (Cyclomethicone) | 72,000 g |
| Seidenpulver | 24,000 g |
| Bentonitgel IPM | 3,000 g |
| Nisin | 1,000 g |
| Imidazol | 6,000 g |
| Parfüm 2 | 2,000 g |

| b) fertiger Spray | |
|---|---|
| Suspensionsgrundlage I | 20,000 g |
| Propan/Butan | 80,000 g |

Die Überlegenheit der vorliegenden Erfindung wird anhand der nachfolgenden Versuche demonstriert. Die Zusammensetzungen gemäß den Beispielen wurden anhand des sogenannten "Sniff-Tests" beurteilt.

### Versuch 1:

Es wurden erfindungsgemäße Zusammensetzungen gemäß Beispiel 1 gegen einen Placebo, also eine bis auf den Gehalt an Wirksubstanz identische Zusammensetzunge getestet.

Ein Kollektiv von 30 Probanden hatte die Auflage, jeweils eine Achselhöhle mit erfindungsgemäßer Zusammensetzung und die andere mit Placebo zu behandeln. Die Probanden trugen daraufhin drei Stunden lang ein Hemd mit Slipeinlagen unter den Achseln. Nach Ablauf dieser Zeit wurden die Slipeinlagen in separate Flaschen überführt. Der Geruch der Einlagen wurde von drei Prüfpersonen beurteilt. Der Versuch wurde als Doppelblindversuch durchgeführt, so daß weder die Probanden noch die Prüfpersonen wußten, welche Achsel mit welcher Zusammensetzung behandelt worden war.

Es stellte sich heraus, daß die wirkstoffhaltigen Zubereitungen in jeweils 29 von 30 Fällen sensorisch beurteilt besser wirkten als die entsprechenden Placebos. In 1 von 30 Fällen gab die Prüfperson an, behandelte und unbehandelte Proben würden sich nicht voneinander unterscheiden.

### Versuch 2:

Es wurden erfindungsgemäße Zusammensetzungen gemäß Beispiel 4 (enthaltend Imidazol, Zubereitungen C) gegen damit identische Zusammensetzungen ohne Imidazol (Zubereitungen D) getestet.

Ein Kollektiv von 30 Probanden hatte die Auflage, jeweils eine Achselhöhle mit Zubereitung C und die andere mit Zubereitung D zu behandeln. Die Probanden trugen daraufhin drei Stunden lang ein Hemd mit Slipeinlagen unter den Achseln. Nach Ablauf dieser Zeit wurden die Slipeinlagen in separate Flaschen überführt. Der Geruch der Einlagen wurde von fünf Prüfpersonen beurteilt. Der Versuch wurde als Doppelblindversuch durchgeführt, so daß weder die Probanden noch die Prüfpersonen wußten, welche Achsel mit welcher Zusammensetzung behandelt worden war.

Es stellte sich heraus, daß die Zubereitungen C in jeweils 25 von 30 Fällen sensorisch beurteilt besser wirkten als die Zubereitungen D.

In je 4 von 30 Fällen wurden die Zubereitungen D sensorisch besser beurteilt als die Zubereitungen C.

In 1 von 30 Fällen gab die Prüfperson an, behandelte und unbehandelte Proben würden sich nicht voneinander unterscheiden.

## Patentansprüche

1. Kombinationen aus Imidazol und/oder einem oder mehreren Derivaten des Imidazols, welche folgende allgemeine Strukturformel aufweisen 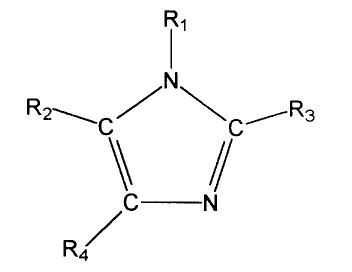
- wobei die Reste folgende Bedeutung haben können:
R₁ = H, C₁₋₂₅ - Alkyl,
R₂ = H, C₁₋₂₅ - Alkyl,
R₃ = H, C₁₋₂₅ - Alkyl,
R₄ = H, C₁₋₂₅ - Alkyl bedeuten,
- wobei R₄, auch, und zwar bevorzugt dann, wenn R₁, R₂ und R₃ ein Wasserstoffatom darstellen, die Reste
-CH₂-CH₂-NR₅R₆ und -CH₂-CH(NR₅R₆)-COOR₇
repräsentieren kann, wobei
R₅ = H, C₁₋₂₅ - Alkyl,
R₆ = H, C₁₋₂₅ - Alkyl,
R₇ = H, C₁₋₂₅ - Alkyl bedeuten,
und einem Lantibiotikum oder einem Gemisch aus mehreren Lantibiotika.

2. Kombinationen nach Anspruch 1, wobei die Lantibiotika gewährt werden aus der Gruppe Nisin, Epidermin, Subtilin, Cinramycin, Duramycin, Ancovenin, Pep 5, Gallidermin.

3. Kombinationen nach Anspruch 1, wobei die Imidazolderivate gewährt werden aus der Gruppe des unsubstituierten Imidazols, des Histidins und des Histamins.

4. Kombination nach Anspruch 1, durch einen Gehart von
0,01 - 99,99 Gew.-% einer Imidazolkomponente und
99,99 - 0,01 Gew.-% eines Einzelstoffes oder eines Gemisches aus der Gruppe der Lantibiotika,
bezogen auf das Gesamtgewicht der Kombination, gekennzeichnet.

5. Zubereitungen, enthaltend Kombinationen nach Anspruch 1, wobei die Lantibiotika in einem Gehart von 0,1 - 10000 ppm, bezogen auf das Gesamtgewicht der Zubereitungen, vorliegen.

6. Zubereitungen, enthaltend Kombinationen nach Anspruch 1, wobei die Lantibiotika in einem Gehalt von 0,1 - 750 ppm, bezogen auf das Gesamtgewicht der Zubereitungen vorliegen.

7. Zinbereitungen, enthaltend Kombinationen nach Anspruch 1, wobei die Lantibiotika in einem Gehalt von 0,5 - 400 ppm, bezogen auf das Gesamtgewicht der Zubereitungen vorliegen.

8. Zubereitungen, enthaltend Kombinationen nach Anspruch 1, wobei das oder die Imidazolderivate in einer Konzentration von 0,01 - 5,00 Gew.-% , bezogen auf das Gesamtgewicht der Zubereitungen vorliegen.

9. Zubereitungen, enthaltend Kombinationen nach Anspruch 1, wobei das oder die Imidazolderivate in einer Konzentration von 0,10 - 2,00 Gew.-% , bezogen auf das Gesamtgewicht der Zubereitungen vorliegen.

10. Zubereitungen, enthaltend Kombinationen nach Anspruch 1, wobei das oder die Imidazolderivate in einer Konzentration von 0,4 - 0,6 Gew.-% , bezogen auf das Gesamtgewicht der Zubereitungen vorliegen.

11. Zubereitungen nach einem der Ansprüche 1 - 10, dadurch gekennzeichnet, daß sie in Form von Deo-Sprays, Roll-ons, Pumpsprays, Tinkturen, Intimreinigungsmittel, Shampoos, Dusch- oder Badezinbereitungen, Pudern, Pudersprays oder Deo-Stifte vorliegen.

12. Verwendung eines oder mehrere Derivate des Imidazols, welche folgende allgemeine Strukturformel aufweisen 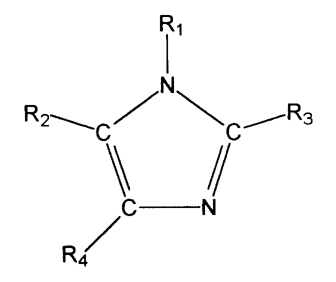
- wobei die Reste folgende Bedeutung haben können:
R₁ = H, C₁₋₂₅ - Alkyl,
R₂ = H, C₁₋₂₅ - Alkyl,
R₃ = H, C₁₋₂₅ - Alkyl,
R₄ = H, C₁₋₂₅ - Alkyl bedeuten,
- wobei R₄, auch, und zwar bevorzugt dann, wenn R₁, R₂ und R₃ ein Wasserstoffatom darstellen, die Reste
-CH₂-CH₂-NR₅R₆ und -CH₂-CH(NR₅R₆)-COOR₇
repräsentieren kann, wobei
R₅ = H, C₁₋₂₅ - Alkyl,
R₆ = H, C₁₋₂₅ - Alkyl,
R₇ = H, C₁₋₂₅ - Alkyl bedeuten,
zur Stabilisierung von Lantibiotika.

13. Verwendung von Kombinationen nach einem der Ansprüche 1 - 3 zur Herstellung einer dermatologischen Zubereitung gegen Akne.

## Claims

1. Combinations of imidazole and/or one or more derivatives of imidazole which have the following general structural formula 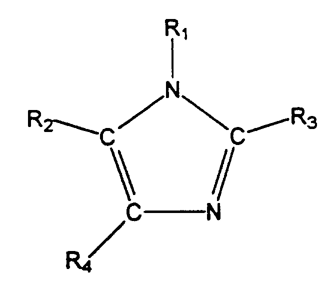
- wherein the radicals can have the following meaning:
R₁ = H or C_{1 - 25} - alkyl,
R₂ = H or C_{1 - 25} - alkyl,
R₃ = H or C_{1 - 25} - alkyl,
R₄ = H or C_{1 - 25} - alkyl,
- wherein R₄ can also, and preferably if R₁, R₂ and R₃ are a hydrogen atom, be the radicals
-CH₂-CH₂-NR₅R₆ and -CH₂-CH(NR₅R₆)-COOR₇,
wherein
R₅ = H or C_{1 - 25} - alkyl,
R₆ = H or C_{1 - 25} - alkyl,
R₇ = H or C_{1 - 25} - alkyl,
and a lantibiotic or a mixture of two or more lantibiotics.

2. Combinations according to Claim 1, wherein the lantibiotics are chosen from the group consisting of nisin, epidermin, subtilin, cinramycin, duramycin, ancovenin, Pep 5 and gallidermin.

3. Combinations according to Claim 1, wherein the imidazole derivatives are chosen from the group consisting of unsubstituted imidazole, histidine and histamine.

4. Combination according to Claim 1, characterized by a content of
0.01 - 99.99 % by weight of an imidazole component and
99.99 - 0.01 % by weight of an individual substance or a mixture from the group of lantibiotics,
based on the total weight of the combination.

5. Formulations comprising combinations according to Claim 1, wherein the lantibiotics are present in a content of 0.1 - 10000 ppm, based on the total weight of the formulations.

6. Formulations comprising combinations according to Claim 1, wherein the lantibiotics are present in a content of 0.1 - 750 ppm, based on the total weight of the formulations.

7. Formulations comprising combinations according to Claim 1, wherein the lantibiotics are present in a content of 0.5 - 400 ppm, based on the total weight of the formulations.

8. Formulations comprising combinations according to Claim 1, wherein the imidazole derivative or derivatives are present in a concentration of 0.01 - 5.00% by weight, based on the total weight of the formulations.

9. Formulations comprising combinations according to Claim 1, wherein the imidazole derivative or derivatives are present in a concentration of 0.10 - 2.00% by weight, based on the total weight of the formulations.

10. Formulations comprising combinations according to Claim 1, wherein the imidazole derivative or derivatives are present in a concentration of 0.4 - 0.6% by weight, based on the total weight of the formulations.

11. Formulations according to one of Claims 1 - 10, characterized in that they are in the form of deodorant sprays, roll-ons, pump sprays, tinctures, intimate cleansing compositions, shampoos, shower or bath formulations, powders, powder sprays or deo sticks.

12. Use of one or more derivatives of imidazole which have the following general structural formula 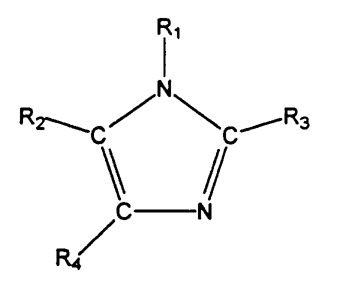
- wherein the radicals can have the following meaning:
R₁ = H or C_{1 - 25} - alkyl,
R₂ = H or C_{1 - 25} - alkyl,
R₃ = H or C_{1 - 25} - alkyl,
R₄ = H or C_{1 - 25} - alkyl,
- wherein R₄ can also, and preferably if R₁, R₂ and R₃ are a hydrogen atom, be the radicals
-CH₂-CH₂-NR₅R₆ and -CH₂-CH(NR₅R₆)-COOR₇,
wherein
R₅ = H or C_{1 - 25} - alkyl,
R₆ = H or C_{1 - 25} - alkyl,
R₇ = H or C_{1 - 25} - alkyl,
for stabilizing lantibiotics.

13. Use of combinations according to one of Claims 1 - 3 for the preparation of a dermatological formulation against acne.

## Revendications

1. Associations d'imidazole et/ou d'un ou plusieurs dérivés de l'imidazole, ayant la formule développée générale suivante 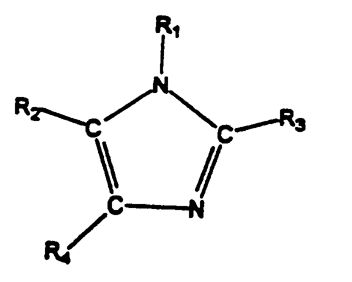
- les radicaux pouvant avoir les significations suivantes:
R₁ représente H ou un groupe alkyle en C₁₋₂₅,
R₂ représente H ou un groupe alkyle en C₁₋₂₅,
R₃ représente H ou un groupe alkyle en C₁₋₂₅,
R₄ représente H ou un groupe alkyle en C₁₋₂₅,
- R₄ pouvant également représenter, et certes de préférence lorsque R₁, R₂ et R₃ représentent un atome d'hydrogène, les radicaux
-CH₂-CH₂-NR₅R₆ et -CH₂-CH(NR₅R₆)-COOR₇,
R₅ représentant H ou un groupe alkyle en C₁₋₂₅,
R₆ représentant H ou un groupe alkyle en C₁₋₂₅,
R₇ représentant H ou un groupe alkyle en C₁₋₂₅,
et d'un lantibiotique ou d'un mélange de plusieurs lantibiotiques.

2. Associations selon la revendication 1, dans lesquelles les lantibiotiques sont choisis dans l'ensemble constitué par la nisine, l'épidermine, la subtiline, la cinramycine, la duramycine, l'ancovénine, Pep 5, la gallidermine.

3. Associations selon la revendication 1, dans lesquelles les dérivés d'imidazole sont choisis parmi l'imidazole non substitué, l'histidine et l'histamine.

4. Association selon la revendication 1, caractérisée par une teneur de
0,01 - 99,99 % en poids en un composant de type imidazole et
99,99 - 0,01 % en poids en une substance individuelle ou un mélange appartenant au groupe des lantibiotiques,
par rapport au poids total de l'association.

5. Préparations contenant des associations selon la revendication 1, dans lesquelles les lantibiotiques sont présents à une concentration de 0,1 - 10 000 ppm, par rapport au poids total des préparations.

6. Préparations contenant des associations selon la revendication 1, dans lesquelles les lantibiotiques sont présents à une concentration de 0,1 - 750 ppm, par rapport au poids total des préparations.

7. Préparations contenant des associations selon la revendication 1, dans lesquelles les lantibiotiques sont présents à une concentration de 0,5 - 400 ppm, par rapport au poids total des préparations.

8. Préparations contenant des associations selon la revendication 1, dans lesquelles le ou les dérivés d'imidazole sont présents à une concentration de 0,01 - 5,00 % en poids, par rapport au poids total des préparations.

9. Préparations contenant des associations selon la revendication 1, dans lesquelles le ou les dérivés d'imidazole sont présents à une concentration de 0,10 - 2,00 % en poids, par rapport au poids total des préparations.

10. Préparations contenant des associations selon la revendication 1, dans lesquelles le ou les dérivés d'imidazole sont présents à une concentration de 0,4 - 0,6 % en poids, par rapport au poids total des préparations.

11. Préparations selon l'une des revendications 1 à 10, caractérisées en ce qu'elles se trouvent sous forme de déodorants à pulvériser, déodorants à appliquer à l'applicateur à bille, compositions à pulvériser au moyen d'un atomiseur, teintures, produits d'hygiène intime, shampooings, préparations pour la douche ou le bain, poudres, poudres à pulvériser ou bâtons déodorants.

12. Utilisation d'un ou plusieurs dérivés de l'imidazole, correspondant à la formule générale développée suivante 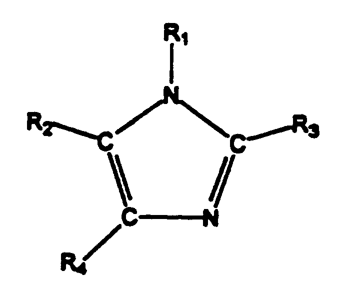
- les radicaux pouvant avoir les significations suivantes:
R₁ représente H ou un groupe alkyle en C₁₋₂₅,
R₂ représente H ou un groupe alkyle en C₁₋₂₅,
R₃ représente H ou un groupe alkyle en C₁₋₂₅,
R₄ représente H ou un groupe alkyle en C₁₋₂₅,
- R₄ pouvant également représenter, et certes de préférence lorsque R₁, R₂ et R₃ représentent un atome d'hydrogène, les radicaux
-CH₂-CH₂-NR₅R₆ et -CH₂-CH(NR₅R₆)-COOR₇,
R₅ représentant H ou un groupe alkyle en C₁₋₂₅,
R₆ représentant H ou un groupe alkyle en C₁₋₂₅,
R₇ représentant H ou un groupe alkyle en C₁₋₂₅,
pour la stabilisation de lantibiotiques.

13. Utilisation d'associations selon l'une des revendications 1 à 3, pour la fabrication d'une préparation dermatologique contre l'acné.
